# EUROPÄISCHE PATENTANMELDUNG

(11) **EP 0 606 590 A1**
(43) Veröffentlichungstag der Anmeldung: **20.07.1994**
(21) Anmeldenummer: 93119914.5
(22) Anmeldetag: 10.12.1993
(51) Int. Cl.: A61K 7/48

(54) **Pharmazeutisches und/oder kosmetisches Mittel, das eine Mischung von N-Acyl-alkanolaminen mit N-Acylphospholipiden oder Lysophospholipiden enthält**

(30) Priorität: 18.12.1992 DE 4242959; 18.12.1992 DE 4242960; 26.01.1993 DE 4302074; 24.02.1993 DE 4305553
(71) Anmelder: RHONE-POULENC RORER GMBH, 50792 Köln (DE)
(72) Erfinder: Ghyczy, Miklos, 50933 Köln (DE); Nissen-Zoufal, Brigitte, 53347 Alfter (DE); Gehring, Wolfgang, 76467 Bietigheim (DE)
(74) Vertreter: Döring, Wolfgang, Dr. Ing.

(57) **Zusammenfassung**

Es wird ein pharmazeutisches und/oder kosmetisches Mittel beschrieben, wobei das Mittel als Wirkstoff neben ggf. üblichen Zusatzstoffen eine Mischung von N-Acyl-Alkanolaminen mit N-Acyl-Ethanolaminen enthält.

## Beschreibung

Die vorliegende Erfindung betrifft ein pharmazeutisches und/oder kosmetisches Mittel.

Die Verwendung von N-Acyl-Ethanolaminen in Produkten zur Haarpflege ist bekannt. So beschreibt beispielsweise eine Veröffentlichung von Goldemberg in J.Soc.Cosmet. Chem., 30, 1979, S. 415 - 427 die Anwendung von Acetyl-Ethanolaminen (MEA), um hierdurch Augenreizungen, hervorgerufen durch tensidische Bestandteile in Shampoos, zu reduzieren.

Die orale Anwendung von N-Oleyl-Phosphatidylethanolaminen als pharmazeutisches Mittel besitzt gemäß der DE 27 56 866 A1 eine deutlich antiarteriosklerotische bzw. antilipämische Wirkung, was anhand von tierexperimentellen Prüfungen bewiesen werden konnte.

Die DE 36 06 664 A1 ist auf ein Pflanzenbehandlungsmittel gerichtet, welches neben einem Fungizid-Wirkstoff noch als weiteren Wirkstoff N-Acyl-Alkanolamin enthält, wobei der weitere Wirkstoff die Wirksamkeit der Fungizide erhöht.

Der vorliegenden Erfindung liegt die Aufgabe zugrunde, ein neues pharmazeutisches und/oder kosmetisches Mittel mit einer besonders hohen Wirksamkeit zur Verfügung zu stellen.

Diese Aufgabe wird erfindungsgemäß durch ein pharmazeutisches und/oder kosmetisches Mittel mit den kennzeichnenden Merkmalen des Patentanspruchs 1 gelöst.

Das erfindungsgemäße Mittel, das sowohl zur pharmazeutischen als auch kosmetischen Anwendung geeignet ist, enthält als Wirkstoff neben ggf. üblichen Zusatzstoffen eine Mischung von N-Acyl-Alkanolaminen der allgemeinen Formel I 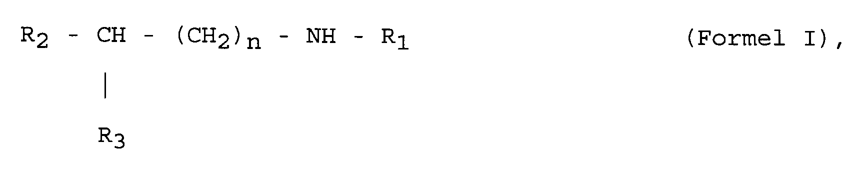
wobei in der Formel I
- R₁: der Acylrest einer gesättigten C₁-C₁₈-Carbonsäure und/oder eines gesättigten C₁-C₁₈-Carbonsäurederivates und/oder
der Acylrest einer ungesättigten C₃ - C₁₈-Carbonsäure und/oder eines ungesättigten C₃ - C₁₈-Carbonsäurederivates;
- R₂: eine Hydroxy-Gruppe und/oder eine Estergruppe, jedoch keine Phosphorsäureestergruppe;
- R₃: Wasserstoff und/oder der C₁-C₄-Alkylrest; und
- n: eine ganze Zahl zwischen 0 und 5 bedeuten,
mit N-Acyl-Ethanolaminen der allgemeinen Formel II 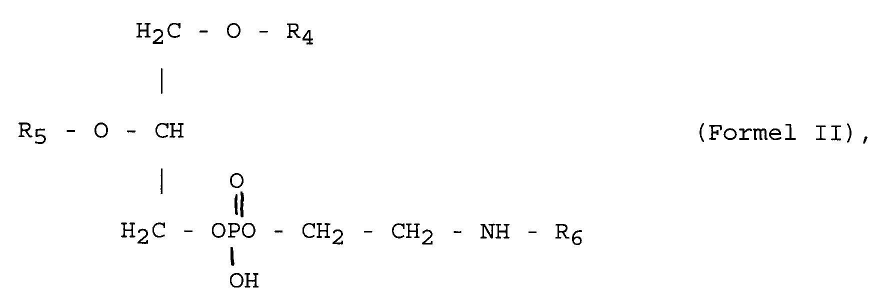
wobei in der Formel II
- R₆: der Acylrest einer gesättigten C₁-C₁₈-Carbonsäure und/oder eines gesättigten C₁-C₁₈-Carbonsäurederivates und/oder
der Acylrest einer ungesättigten C₃ - C₁₈-Carbonsäure und/oder eines ungesättigten C₃ - C₁₈-Carbonsäurederivates;
- R₅: Wasserstoff und/oder der Acylrest einer gesättigten und/oder ungesättigten C₁₆ - C₁₈-Carbonsäure und/oder der Acylrest einer gesättigten und/oder ungesättigten C₁₆-C₁₈-Carbonsäurederivates; und
- R₄: Wasserstoff und/oder der Acylrest einer gesättigten und/oder ungesättigten C₁₆-C₁₈-Carbonsäure und/oder der Acylrest eines C₁₆-C₁₈-Carbonsäurederivates sind.

Überraschend konnte festgestellt werden, daß die zuvor angegebene Mischung der Wirkstoffe gemäß der Formel I und gemäß der Formel II hervorragende pharmazeutische und/oder kosmetische Eigenschaften aufweisen, die sich insbesondere beispielsweise darin ausdrücken, daß sich derartige Mittel hervorragend zur Verhinderung und insbesondere auch zur Behandlung von Hautschäden und/oder Hauterkrankungen, wie beispielsweise Verbrennungen, Aknen, Insektenstichen, Herpes-Infektionen o.dgl., einsetzen lassen. Weiterhin weisen derartige Mittel den weiteren Vorteil auf, daß hierin Wirkstoffe enthalten sind, die toxikologisch völlig unbedenklich sind, zumal die in den Formeln I und II wiedergegebenen Wirkstoffe im menschlichen Körper ohnehin vorkommen. Ferner lassen sich die zuvor genannten Wirkstoffe in wäßrigen Systemen lösen oder stabil dispergieren bzw. emulgieren, so daß die Herstellung eines lagerstabilen kosmetischen bzw. pharmazeutischen Mittels keine Probleme bereitet. Auch ist die Verfügbarkeit der in den Formeln I und II allgemein beschriebenen Wirkstoffe gegeben, wobei vorzugsweise die insbesondere in der Formel II wiedergegebenen Phosphorsäureester aus Naturprodukten, vorzugsweise Soja-Phospholipiden, isoliert werden können.

Die Konzentration der durch die Formeln I und II wiedergegebenen Wirkstoffmischung in dem erfindungsgemäßen pharmazeutischen bzw. kosmetischen Mittel richtet sich nach der Art der medizinischen Indikation des Mittels. Üblicherweise variiert die Konzentration der Wirkstoffmischung in dem erfindungsgemäßen Mittel zwischen 0,5 Gew.% und 25 Gew.%, vorzugsweise 1 Gew.% und 10 Gew.%, bezogen jeweils auf die Masse des anwendungsfertigen Mittels.

Bezüglich des Massenverhältnisses der N-Acyl-Alkanol-Amins (Formel I) zum N-Acyl-Ethanol-Amin (Formel II) ist festzuhalten, daß dieses Massenverhältnis zwischen 55:45 und 80:20 variiert.

Besonders hervorragende Eigenschaften bezüglich der Verhinderung bzw. der Behandlung der vorstehend angegebenen und nachfolgend noch aufgeführten Schädigungen, Reizungen bzw. Erkrankungen weist eine Ausführungsform des erfindungsgemäßen Mittels auf, bei dem das N-Acyl-Alkanol-Amin einen chemischen Aufbau besitzt, wie dieser durch die Formel III wiedergegeben ist. 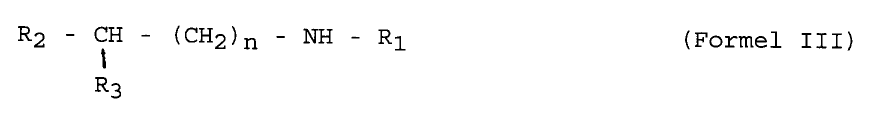
In der Formel III bedeuteten
- R₁: der Acylrest der Essigsäure, der Propionsäure, der 2-Hydroxypropionsäure, der Palmitinsäure, der Stearinsäure und/oder der Ölsäure;
- R₂: eine Hydroxy-Gruppe;
- R₃: Wasserstoff und/oder eine Methylgruppe; und
- n: 1 oder 2.

Insbesondere bietet es sich an, die nachfolgend aufgeführten konkreten Derivate von Verbindungen gemäß der Formel III oder Mischungen hiervon zu verwenden:

Essigsäure-2-Hydroxy-Ethylamid, Laurinsäure-2-Hydroxy-Ethylamid, Stearinsäure-2-Hydroxy-Ethylamid, Linolsäure-2-Hydroxy-Ethylamid, Palmitinsäure-2-Hydroxy-Ethylamid, Laurinsäure-2-Hydroxy-Propylamid, Linolsäure-2-Hydroxy-Propylamid, Ölsäure-2-Hydroxy-Propylamid und die entsprechenden 2-Hydroxy-Ethylamid- und/oder 2-Hydroxy-Propylamid-Derivate, die als Acylreste Fettsäuren aus Kokosfett (Kokosöl) und/oder Palmöl enthalten.

Ebenfalls eine besonders hohe pharmazeutische Wirksamkeit besitzt eine andere Ausführungsform des erfindungsgemäßen Mittels, bei dem das N-Acyl-Ethanolamin einen chemischen Aufbau besitzt, wie dieser durch die nachfolgende Formel IV wiedergegeben ist. 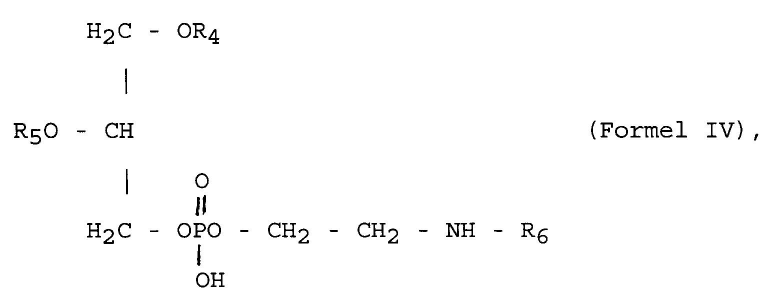
In der vorstehend aufgeführten Formel IV bedeuten
- R₄ und R₅,: die gleich oder verschieden sind, Wasserstoff und/oder der Acylrest der Palmitinsäure, der Stearinsäure, der Linolsäure, der Linolensäure und/oder der Ölsäure;
und
- R₆: der Acylrest der Essigsäure, der Propionsäure, der 2-Hydroxy-Propionsäure, der Palmitinsäure, der Stearinsäure und/oder der Ölsäure.

Vorzugsweise wird das vorstehend durch die Formel IV wiedergegebene N-Acyl-Ethanolamin aus natürlichem Material, insbesondere aus Pflanzenlecithinen, so zum Beispiel Sojabohnen, isoliert. Besonders gute Ergebnisse lassen sich dann erzielen, wenn als Wirkstoff N-Acetyl-Phosphatidylethanolamin, N-Palmityl-Phosphatidylethanolamin und/oder N-Oleyl-Phosphatidylethanolamin jeweils allein oder in Mischung eingesetzt wird.

Insbesondere dann, wenn das erfindungsgemäße Mittel einen Wirkstoff aufweist, der aus N-Acetyl-Ethanolamin, N-Oleoyl-Ethanolamin, N-Linolenoyl-Ethanolamin, N-Acyl-2-Hydroxy-Propylamid und/oder N-Acyl-Ethanolamin, wobei die zuletzt genannten beiden Verbindungen als Acylreste Fettsäuren aus Kokosfett (Kokosöl) und/oder Palmöl enthalten, mit N-Acetylphosphatidylethanolamin besteht, weist ein derartiges Mittel hervorragende prophylaktische und therapeutische Effekte auf, insbesondere in bezug auf die vorstehend wiedergegebenen und nachfolgend noch beschriebenen Schädigungen, Reizungen und/oder Erkrankungen der Haut.

Wie bereits vorstehend ausgeführt ist, kann das erfindungsgemäße Mittel in jeder Darreichungsform, so z.B. als Tabletten, Zäpfchen oder als Infusionslösung, aufgemacht werden. Besonders geeignet ist es jedoch, wenn das erfindungsgemäße Mittel eine Darreichungsform besitzt, die eine topische, ggf. lokale, Anwendung gestattet. Dies bedeutet, daß in diesem Fall das erfindungsgemäße pharmazeutische bzw. kosmetische Mittel die für die topische Anwendung üblichen Zusatzstoffe enthält und vorzugsweise als flüssige, halbfeste oder feste Zubereitung vorliegt.

Für die flüssige Zubereitung kommen Tropfen, Tinkturen oder Sprays in Frage, die die zuvor beschriebenen Wirkstoffmischungen in Form einer Lösung, Suspension, Emulsion oder Dispersion enthalten.

Als halbfeste Zubereitungen kommen beispielsweise Gele, Salben, Cremes oder Schäume in Frage, während unter feste Zubereitungen beispielsweise Pulver, Puder, Granulate, Pellets oder Mikrokapseln fallen. Werden die zuvor beschriebenen Wirkstoffmischungen in der Form einer flüssigen Darreichungsform als pharmazeutisches und/oder kosmetisches Mittel angeboten, so empfiehlt es sich, hierfür möglichst solche Verdünnungsmittel zu verwenden, die die Haut bei einer topischen Anwendung nicht reizen. Dies trifft beispielsweise insbesondere auf Wasser, einwertige Alkohole, vorzugsweise Ethanol, Isopropanol oder n-Propanol, mehrwertige Alkohole, insbesondere Glycerin und/oder Propandiol, Polyglykole, insbesondere Polyethylenglykole und/oder Miglyol, Glycerinformal, Dimethylisosorbit, natürliche und synthetische Öle und/oder Ester, zu.

Für die Herstellung von halbfesten Darreichungsformen, wie beispielsweise Gele, Salben, Cremes und Schäume, eignen sich neben den zuvor genannten Verdünnungsmitteln zusätzlich noch Grundmassen, wie beispielsweise Bentonit, Veegum, Guarmehl und/oder Cellulosederivate, insbesondere Methylcellulose und/oder Carboxymethylcellulose. Ebenso kommen als Grundmasse anstelle der zuvor genannten Grundmassen oder zusätzlich zu den zuvor genannten Grundmassen Polymere aus Vinylalkohole, Vinylpyrolidone, Alginate, Pektine, Polyacrylate, feste und/oder flüssige Polyethylenglykole, Paraffine, Fettalkohole, Vaselin, Wachse, Fettsäuren und/oder Fettsäureester in Frage.

Für die Herstellung von festen, ebenfalls zur topischen Anwendung geeigneten Zubereitungen, wie beispielsweise die zuvor genannten Pulver, Puder, Granulate, Pellets oder Mikrokapseln, besteht die Möglichkeit, hier als Bindemittel beispielsweise kolloidale Kieselsäure, Talkum, Milchzucker, Stärkepulver, Zucker, Cellulosederivate, Gelatine, Metalloxide und/oder Metallsalze zu verwenden, wobei die Konzentration des Wirkstoffgemisches und das Massenverhältnis der mindestens beiden Wirkstoffe (Formel I, Formel III bzw. Formel II, Formel IV) innerhalb der eingangs genannten Bereiche variieren.

Darüber hinaus kann das erfindungsgemäße Mittel wahlweise noch weitere Bestandteile, wie beispielsweise Konservierungsmittel, Stabilisatoren, Tenside, Emulgatoren, Penetrationsförderer, Spreitungsmittel und/oder Treibmittel, enthalten.

Eine besonders geeignete Ausführungsform des erfindungsgemäßen Mittels, die insbesondere zur topischen Anwendung geeignet ist, weist als weiteren Bestandteile Phospholipide, insbesondere Soja-Phospholipide, auf. Hierfür werden vorzugsweise solche Soja-Phospholipidfraktionen verwendet, die mindestens 80 Gew.% Phosphatidylcholin enthalten, wobei die Konzentration des phospholipidischen Bestandteils in dem anwendungsfertigen erfindungsgemäßen Mittel je nach Zubereitungsform und Anwendung zwischen 1 Gew.% und 25 Gew.%, vorzugsweise zwischen 2 Gew.% und 15 Gew.%, variiert.

Wie bereits eingangs kurz erwähnt, wird das erfindungsgemäße Mittel insbesondere als topisch anzuwendendes pharmazeutisches oder kosmetisches Mittel zur Verhinderung von Hautschäden und/oder Hauterkrankungen, die durch Lichtstrahlung, insbesondere Sonnenstrahlung, herbeigeführt werden, eingesetzt. Überraschend konnte festgestellt werden, daß das erfindungsgemäße Mittel wirksam Lichtdermatosen und lichtbeeinflußbare Dermatosen, wie beispielsweise Dermatitis solaris, aktinische Atrophie, Cutis rhomboidalis nuchae, Erythrosis interfollicularis colli, Morbus Favre Racouchot, Keratosen, Cornu cutaneum, Cheilitis actinica, Sommerprurigo, Mallorca-Akne, phototoxische Dermatitis, Wiesengräserdermatitis, photoallergische Dermatitis, polymorphe Lichtdermatosen, Hydroa racciniformia, Xeroderma pigmentosum, Lupus erythematodes, rosaceaartige Dermatitis, seborrhoische Ekzeme und Lichen ruber planus, sowie die bei den zuvor genannten Dermatosen auftretenden Begleiterscheinungen, wie insbesondere das Austrocknen, Rauhwerden, die Rötung und die Faltenbildung sowie das vorzeitige Altern der Haut, verhindert, so daß das erfindungsgemäße Mittel sich insbesondere auch als Sonnenschutzmittel und somit zur Prophylaxe einsetzen läßt.

Ferner konnte überraschend festgestellt werden, daß sich das erfindungsgemäße Mittel auch hervorragend zur Therapie von durch Licht und/oder Wärme hervorgerufenen Hautschäden, insbesondere auch der zuvor genannten Hautschäden, in der topischen Anwendung einsetzen läßt, so daß eine rasche Heilung auftritt.

Auch bei Insektenstichen, so zum Beispiel Bienenstichen, Wespenstichen, Hornissenstichen, Mückenstichen, Milbenstichen, Zeckenbissen, Sandflohbissen, Stechfliegenbissen und Bremsenbissen, konnte festgestellt werden, daß bereits nach kürzester Zeit nach Auftragen des erfindungsgemäßen Mittels ein deutlicher Rückgang der durch die zuvor genannten Stiche bzw. Bisse hervorgerufenen Rötungen, Schwellungen und/oder Quaddelbildungen auftritt, während gleichzeitig der durch den Insektenstich bzw. Insektenbiß hervorgerufene Schmerzreiz bzw. Juckreiz deutlich reduziert wird.

Selbst bei sehr empfindlichen Patienten konnte bei einer topischen Anwendung des erfindungsgemäßen Mittels keine unerwünschte Reizung oder Rötung der Haut festgestellt werden.

Wird das beanspruchte pharmazeutische bzw. kosmetische Mittel zur Verhinderung von durch Lichtstrahlung, insbesondere Sonnenstrahlen, verursachten Hauterkrankungen bzw. Hautschäden verwendet, so empfiehlt es sich insbesondere in den Fällen, in denen das Mittel einen besonders hohen Lichtschutzfaktor aufweisen soll, zusätzlich noch zu der Wirkstoffmischung eine Substanz zuzusetzen, die eine lichtstrahlenabsorbierende oder lichtstrahlenreflektierende Wirkung besitzt. Hierfür kommen insbesondere solche Substanzen in Frage, die auf der Basis von p-Aminobenzoesäure, Octyldimethyl-p-Aminobenzoesäure, Anthranilate, Cinnamate, Benzophenone, Campherderivate, Zinkoxid und/oder Titandioxid aufgebaut sind, wobei jedoch im Unterschied zu den bekannten Sonnenschutzmitteln die Konzentration dieser lichtabsorbierenden bzw. lichtreflektierenden Substanzen vorzugsweise erheblich im Vergleich zu bekannten Sonnenschutzmitteln reduziert wird, so insbesondere auf eine Konzentration, die zwischen 50 % und 70 % unterhalb der Konzentration liegt, die diese Substanzen in den bekannten Sonnenschutzmitteln ausmachen. Dies hängt vermutlich damit zusammen, daß das in dem erfindungsgemäßen Mittel vorhandene Wirkstoffgemisch, bestehend aus N-Acyl-Alkanolaminen und N-Acyl-Ethanolaminen der vorstehend genannten Art, offensichtlich mit den bekannten lichtabsorbierenden bzw. lichtreflektierenden Substanzen eine synergistische Wirkung besitzt, so daß diese bekannten lichtabsorbierenden bzw. lichtreflektierenden Substanzen unter Beihaltung eines identischen Lichtschutzfaktors erheblich reduziert werden können.

Durch die in der vorliegenden Anmeldung gewählte Formulierung und/oder bei den vorstehend wiedergegebenen Bedeutungen für R₁, R₂, R₃, R₄ und R₅ soll zum Ausdruck gebracht werden, daß die jeweils eingesetzten N-Acyl-Alkanolamine (Formel I, Formel III) bzw. die N-Acyl-Ethanolamine (Formel II, Formel IV) keine chemisch einheitlich aufgebauten Verbindungen sein müssen. So können diese Verbindungen Mischungen darstellen, die beispielsweise eine Vielzahl von Einzelverbindungen enthalten, die sich bezüglich des Acylrestes, der Hydroxy-Gruppe, der Estergruppe und/oder der Bedeutung von R₃ unterscheiden.

Abhängig von der jeweiligen Anwendung kann die Zubereitung noch hautpflegende Zusätze oder solche Bestandteile enthalten, die die therapeutische Wirksamkeit erhöhen. Hierfür kommen insbesondere Lokalanästhetika, Antibiotika, Antimykotika, Antihistaminika, Steroide, UV-Filter, Vitamine, Peptide, Kollagene, Hyaluronsäure und/oder Pflanzenextrakte in Frage.

Vorteilhafte Weiterbildungen des erfindungsgemäßen Mittels sind in den Unteransprüchen angegeben.

Das erfindungsgemäße Mittel wird nachfolgend anhand von Ausführungsbeispielen näher beschrieben.

### Ausführungsbeispiel 1

Es wurde ein Produkt 1 hergestellt, wobei das Produkt 1 die nachfolgend genannten Bestandteile aufwies:

| | | |
|---|---|---|
| Phase A | 55,00 g | Wasser |
| | 14,00 g | Propylenglycol |
| | 18,00 g | Phosphatidylcholin (mindestens 80 Gew.% Soja-Phosphatidylcholin) |
| Phase B | 1,00 g | N-Acetylphosphatidylethanolamin |
| | 2,00 g | N-Acetylethanolamin |
| | 10,00 g | Jojobaöl |

Die Phase A wurde bei 40 °C in einem schnell laufenden Rührer 40 Minuten lang bis zur Ausbildung eines klaren Geles homogenisiert. Nach Zufügen der Phase B wurde so lange weitergerührt, bis sich beide Phasen unter Ausbildung eines klaren Geles homogenisiert hatten.

### Ausführungsbeispiel 2

Es wurde ein Produkt 2 aus den nachfolgend wiedergegebenen Bestandteilen hergestellt.

| | | |
|---|---|---|
| Phase A | 52,00 g | Wasser |
| | 14,00 g | Propylenglykol |
| | 18,00 g | Phosphatidylcholin (wie bei Produkt 1) |
| Phase B | 2,00 g | N-Acetylphosphatidylethanolamin |
| | 4,00 g | N-Acetylethanolamin |
| | 10,00 g | Jojobaöl |

Die Phase A wurde bei 40 °C in einem schnell laufenden Rührer 40 Minuten unter Ausbildung eines klaren Geles homogenisiert.

Anschließend wurde die Phase B hinzugefügt und so lange gerührt, bis beide Phasen homogenisiert waren.

### Ausführungsbeispiel 3

Es wurde ein drittes Produkt hergestellt, daß die folgenden Bestandteile aufwies.

| | | |
|---|---|---|
| Phase A | 58,00 g | Wasser |
| | 14,00 g | Propylenglykol |
| | 18,00 g | Phosphatidylcholin |
| Phase B | 0,00 g | N-Acetylphosphatidylethanolamin |
| | 0,00 g | N-Acetylethanolamin |
| | 10,00 g | Jojobaöl |

Die Phase A wurde bei 40 °C in einem schnell laufenden Rührer während 40 Minuten unter Ausbildung eines klaren Geles homogenisiert. Nach Zugabe der Phase B wurde so lange gerührt, bis beide Phasen homogen vermischt waren.

Die zuvor genannte Produkte 1 bis 3 wurden zur dermatologischen Bewertung des Erythemschutzes von externen Sonnenschutzmitteln für die menschliche Haut einer Prüfung nach DIN 67 501 unterzogen, wobei diese Prüfung an 20 Probanden durchgeführt wurde.

Die Prüfung ergab folgende Werte:

| Produkt | mittlerer gefundener Lichtschutzfaktor (LSF) |
|---|---|
| Produkt 1 | 8,7 |
| Produkt 2 | 9,5 |
| Produkt 3 | 2,1 |

Um die heilende Wirkung des Produktes 1 in bezug auf Entzündungen zu beweisen, wurde eine Untersuchung an 10 gesunden Probanden durchgeführt.

Hierbei wurde eine Entzündung mittels subkutaner Applikation von Histamin ausgelöst, die dabei auftretende Rötung mittels Chromameter und die dabei auftretende Hautdurchblutung mittels Laser-Doppler-Methode quantifiziert. Diese Methode ist insoweit für die Überprüfung der heilenden Wirkung von Lichtschädigungen relevant, da die primäre Schädigung der Haut durch Licht die Freisetzung des Histamins aus den Mastzellen der Haut bewirkt. Das dabei freigesetzte Histamin seinerseits führt wiederum zu Hautentzündungen, wie diese beispielsweise auch beim Sonnenbrand auftreten.

Die zuvor beschriebene Testmethode stellt eine anerkannte Testmethode dar und ist in "Methods in Skin Research, 1985, S. 511 ff" beschrieben.

Der Verlauf der Heilung des Entzündungsvorganges wird durch die nachfolgenden beiden Abbildungen 1 und 2 wiedergegebenen.

Wie die beiden Abbildungen 1 und 2 eindeutig beweisen, zeigt das Produkt 1 bereits nach 15 bzw. 30 Minuten im Vergleich zum Produkt 3 eine signifikante Abnahme der Rötung und der Hyperämie.

In einem weiteren Test wurde quantifiziert, inwieweit β-Karotin zerstört wird, das auf die Haut aufgetragen wurde. β-Karotin stellt eine der wichtigsten Verbindungen dar, um lebendes Gewebe gegen eine Zerstörung, die durch Licht ausgelöst wird, zu schützen. Hierbei wurde auf den Unterarm von 10 Probanden zunächst 40 µl einer β-Karotin-Lösung (5 mg in 2,5 ml Hexan) auf eine definierte große markierte Hautstelle aufgetragen. Nach einer Verweilzeit von 10 Minuten wurden die Produkte 2 und 3 auf die definierten Hautstellen aufgebracht, wobei die Konzentration am Produkt 3 mg/cm² betrug.

Die so behandelten definiert großen Hautflächen wurden nach einer Stunde mit einer Ultra-Vita-UVL-Lampe 3 Minuten lang in einem Abstand von 50 cm bestrahlt. Unmittelbar danach wurde die Hautfläche mit 1 ml Isopropanol 1 Minute lang extrahiert. In dem Extrakt wurde das β-Karotin hochdruckflüssigkeitschromatographisch quantitativ bestimmt. Hierfür wurde Methanol/n-Hexan (V:V 75:25) als Eluent bei einer Flow-Rate von 1 ml/min unter Anwendung einer RP 18,3 µ 4,6 x 75 mm (Ultrasphere Beckman-Säule) eingesetzt. Am Auslaß der Säule war ein UV-Detektor vorgesehen, der auf eine Wellenlänge von 436 nm eingestellt war.

Die Zerstörung des β-Karotins infolge der Lichteinstrahlung geht aus der nachfolgenden Tabelle hervor.

| Produkt | relatives prozentuales Maß der Zerstörung |
|---|---|
| Produkt 2 | 24 % |
| Produkt 3 | 100 % |

In einer Fallstudie an 10 Probanden, die Verbrennungen 1. und 2. Grades zeigten, wurde die Wirksamkeit des Produktes 2 im Vergleich zum Produkt 3 bewiesen. Hierbei wurden ausgewählte Verbrennungsbereiche mit einer Hautgröße von 4 cm² markiert und jeweils mit Produkt 2 oder Produkt 3 behandelt. Der zeitliche Verlauf der Heilung zeigte eindeutig, daß bei den Hautbereichen, die mit dem Produkt 2 behandelt wurden, die Zeit bis zur erfolgten Heilung 30 % bis 60 % kürzer war als bei den Bereichen, die mit dem Produkt 3 behandelt worden sind.

In einer weiteren Fallstudie wurde die Wirksamkeit des Produktes 2 am Beispiel von Insektenstichen untersucht. Hierbei wurde bei 4 Probanden, die auf den Armen bzw. den Beinen offensichtlich zeitgleich drei bis sechs Mückenstiche erhalten hatten, ein Teil dieser Mückenstiche mit dem Produkt 2 und ein anderer Teil der Mückenstiche mit dem Produkt 3 behandelt. Bei dem mit dem Produkt 2 behandelten Mückenstichen zeigte sich bereits nach einer kurzen Zeit (15 - 30 Minuten) ein deutlicher Rückgang der Rötung und Schwellung sowie ein Nachlassen des Juckreizes, während die mit Produkt 3 behandelten Mückenstiche auch noch nach 2 bis 4 Stunden deutlich gerötet und erhaben waren und den unangenehmen Juckreiz zeigten.

## Patentansprüche

1. Pharmazeutisches und/oder kosmetisches Mittel, dadurch gekennzeichnet, daß es als Wirkstoff neben ggf. üblichen Zusatzstoffen eine Mischung von N-Acyl-Alkanolaminen der allgemeinen Formel I 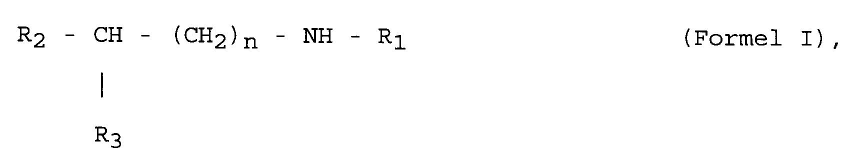 wobei in der Formel I
R₁ der Acylrest einer gesättigten C₁-C₁₈-Carbonsäure und/oder eines gesättigten C₁-C₁₈-Carbonsäurederivates und/oder
der Acylrest einer ungesättigten C₃ - C₁₈-Carbonsäure und/oder eines ungesättigten C₃ - C₁₈-Carbonsäurederivates;
R₂ eine Hydroxy-Gruppe und/oder eine Estergruppe, jedoch keine Phosphorsäureestergruppe;
R₃ Wasserstoff und/oder ein C₁ - C₄-Alkylrest; und
n eine ganze Zahl zwischen 0 und 5 bedeuten,
mit N-Acyl-Ethanolaminen der allgemeinen Formel II, 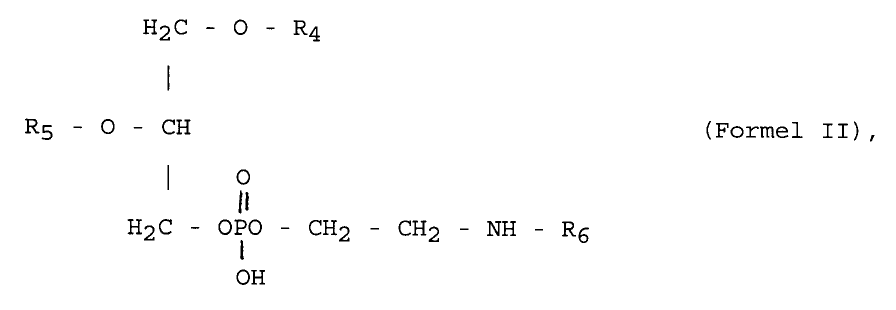 wobei in der Formel II
R₆ der Acylrest einer gesättigten C₁-C₁₈-Carbonsäure und/oder eines gesättigten C₁-C₁₈-Carbonsäurederivates und/oder
der Acylrest einer ungesättigten C₃ - C₁₈-Carbonsäure und/oder eines ungesättigten C₃ - C₁₈-Carbonsäurederivates;
R₅ Wasserstoff und/oder der Acylrest einer gesättigten und/oder ungesättigten C₁₆ - C₁₈-Carbonsäure und/oder der Acylrest eines gesättigten und/oder ungesättigten C₁₆ - C₁₈-Carbonsäurederivates; und
R₄ Wasserstoff und/oder der Acylrest einer gesättigten und/oder ungesättigten C₁₆ - C₁₈-Carbonsäure und/oder der Acylrest eines gesättigten und/oder ungesättigten C₁₆ - C₁₈-Carbonsäurederivates bedeuten.

2. Mittel nach Anspruch 1, dadurch gekennzeichnet, daß es die Mischung in einer Konzentration zwischen 0,5 Gew.% und 25 Gew.%, vorzugsweise in einer Konzentration zwischen 1 Gew.% und 10 Gew.%, bezogen auf die Masse des anwendungsfertigen Mittels enthält.

3. Mittel nach Anspruch 1 oder 2, dadurch gekennzeichnet, daß es die Wirkstoffmischung in einem Massenverhältnis von N-Acyl-Alkanolamin:N-Acyl-Ethanolamin zwischen 55:45 und 80:20 aufweist.

4. Mittel nach einem der vorangehenden Ansprüche, dadurch gekennzeichnet, daß das N-Acyl-Alkanolamin einen chemischen Aufbau aufweist, wie dieser durch Formel III wiedergegeben ist, 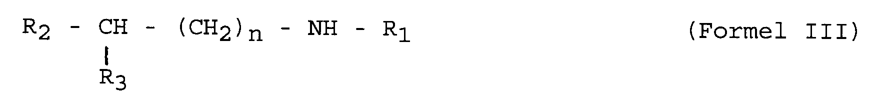 wobei in Formel III
R₁ der Acylrest der Essigsäure, der Propionsäure, der 2-Hydroxypropionsäure, der Palmitinsäure, der Stearinsäure und/oder der Ölsäure;
R₂ eine Hydroxy-Gruppe;
R₃ Wasserstoff und/oder eine Methylgruppe; und
n 1 oder 2 bedeuten.

5. Mittel nach einem der vorangehenden Ansprüche, dadurch gekennzeichnet, daß das N-Acyl-Ethanolamin einen chemischen Aufbau aufweist, wie dieser durch die Formel IV wiedergegeben ist, 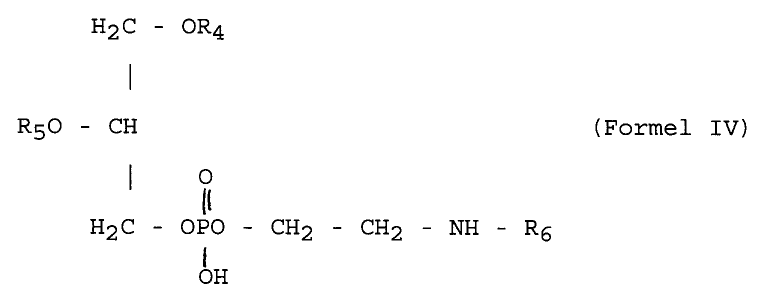 wobei in der Formel IV
R₄ und R₅ gleich oder verschieden sind und den Acylrest der Palmitinsäure, der Stearinsäure, Linolsäure, Linolensäure und/oder der Ölsäure;
und
R₆ den Acylrest der Essigsäure, der Propionsäure, der 2-Hydroxy-Propionsäure, der Palmitinsäure, der Stearinsäure, Linolsäure, Linolensäure und/oder der Ölsäure
darstellen.

6. Mittel nach einem der vorangehenden Ansprüche, dadurch gekennzeichnet, daß der Wirkstoff aus der Mischung von N-Acetyl-Phosphatidylethanolamin mit N-Acetyl-Ethanolamin, N-Oleoyl-Ethanolamin, N-Linolenoyl-Ethanolamin, N-Acyl-Ethanolamin und/oder N-Acyl-2-Hydroxy-Propylamin besteht, wobei die zuletzt genannten beiden Verbindungen als Acylreste Fettsäuren aus Kokosfett und/oder Palmöl enthalten.

7. Mittel nach einem der vorangehenden Ansprüche, dadurch gekennzeichnet, daß es eine für die topische Anwendung geeignete Darreichungsform besitzt und die hierfür üblichen Zusatzstoffe enthält.

8. Mittel nach Anspruch 7, dadurch gekennzeichnet, daß es als weitere Bestandteile Phospholipide, insbesondere Soja-Phospholipide, enthält.

9. Verwendung des Mittels nach einem der vorangehenden Ansprüche zur Verhinderung von durch Lichtstrahlung, insbesondere Sonnenstrahlung, verursachten Hautschäden.

10. Verwendung des Mittels nach einem der Ansprüche 1 bis 8 zur Behandlung von durch Licht und/oder Wärme oder von durch Insektenstichen hervorgerufenen Hautschäden.
